# EUROPEAN PATENT APPLICATION

(11) **EP 2 034 013 A1**
(43) Date of publication of application: **11.03.2009**
(21) Application number: 06769367.1
(22) Date of filing: 21.06.2006
(51) Int. Cl.: C12N 9/64, A61K 38/48, C12N 5/00, A01H 4/00

(54) **PROCESS FOR THE PRODUCTION OF BROMELAIN BY MEANS OF SUBSTANCES THAT INDUCE PROTEINS IN PINEAPPLE PLANTS**

(71) Applicant: Ortiz Barba, Zazil, Nayarit (MX)
(72) Inventor: Ortiz Barba, Zazil, Nayarit (MX)
(74) Representative: Elzaburu Marquez, Alberto
(86) International application number: PCT/MX2006/000055
(87) International publication number: WO 2007/148951

(57) **Abstract**

The present invention relates to the field of biotechnology, and in particular to obtaining secondary metabolites, via induction, such as bromelain from plants of the genus *Ananas* spp., previously established *in vitro.* The subject of the invention is the developments of an innovative process for extracting bromelain from the natural tissues of the pineapple *(Ananas comosus* (L) Merr) plant that have been established and induced *in vitro* with a higher proteolytic activity (200 to 600%) - and in high concentrations - as compared with the protein generated naturally in the plant and currently extracted using conventional methodologies. In this process, use is made of pineapple seedlings established and induced *in vitro,* which are macerated in a phosphate buffer for the purposes of their extraction. Later, they are subjected to filtering, centrifugation, freezing and lyophilization processes in order thus to obtain a crude bromelain extract **characterized by** high protein yields and specific activity.

## Description

### FIELD OF THE INVENTION

The present invention relates to the field of biotechnology, and in particular to obtaining secondary metabolites, via induction, such as bromelain from plants of the genus *Ananas* spp., previously established *in vitro.*

### OBJECT OF THE INVENTION

The object of the invention is the development of an innovative process for extracting bromelain from the natural tissues of the pineapple plant that have been established and induced *in vitro,* with a higher proteolytic activity and in high concentrations - as compared to the protein generated naturally in the plant and currently extracted using conventional methodologies.

In this process, use is made of pineapple seedlings established and induced *in vitro,* which are macerated in a phosphate buffer for the purposes of their extraction. Later, they are subjected to filtering, centrifugation, freezing and lyophilization processes in order thus to obtain a crude bromelain extract characterized by protein yields and specific activity.

### BACKGROUND

Currently, there are foods called nutraceuticals, that in addition to their nutritious value provide health benefits, as is the case of the pineapple *(Ananas comosus* L.) which is a mixture of *cysteine* proteases, the most abundant of which is bromelain, a protein that offers beneficial effects on illnesses such as cancer and coronary thrombosis (Mackay, et al., 2003).

As a nutraceutical, bromelain has several pharmacological actions, such as inhibiting plaque aggregation and increasing the absorption of other medications. Bromelain is well absorbed in oral form and clinical and pharmacological evidence indicate that its therapeutic effects improve when administered in high doses. Although the mechanism of its action it is not yet fully understood, it has been shown to be an effective and safe food supplement (Mackay et al., 2003).

Bromelain may help in the treatment of coronary thrombosis that is characterized by a blockage of blood vessels by clots made of a protein called fibrin and that is responsible for at least half the deaths in developed countries such as Great Britain. Heart attacks are frequently caused by a blockage in the blood vessels that irrigate the heart. A similar situation is observed in cerebral vascular accidents (Felton, 1980).

The formation of blood clots is a process that naturally occurs in normal persons but that is controlled by a delicate balance between the formation of clots and their degradation. Bromelain appears to selectively promote the natural degradation of blood clots, without causing hemorrhaging in subjects that experience blood vessel blockages. The blood contains a natural protease that degrades clots called plasmin, which must be activated from its inactive form, plasminogen. If this natural system is not balanced, the level of plasmin may be low, allowing clots to persist and to block blood vessels. Research performed by Steven Tanssing in Hawaii, have shown that bromelain may stimulate the conversion of plasminogen into plasmin, which allows for the efficient destruction of fibrin clots (Fernandez, 2001).

Bromelain may also present an "antithrombotic" property through which the conversion of protrombin into thrombin is blocked, reducing in this way the formation of clots. The anti-inflammatory effect of bromelain is related to this system. It has been suggested that bromelain is a better anti-inflammatory drug that the non-steroidal drugs, such as the aspirin. While aspirin inhibits the synthesis of all prostaglandins, bromelain is more selective and inhibits the production of only those that increase inflammation, with affecting the ones that are anti-inflammatory. In turn, prolonged trauma and stress tend to move the balance towards proinflammatory prostaglandins, a process that is counteracted by bromelain that tends to reestablish the loss of balance (Fernandez, 2001).

Bromelain is used in industrial applications as a meat tenderizer, for dessert preparation, low calorie gelatins, and in the process for manufacturing beer, among others. It is also used in medical applications due to its being a protein whose enzymatic activity makes it beneficial in diseases such as coronary thrombosis, gastric and intestinal ulcers as an immune system modulator and in cancer treatment.

Bromelain is a protease that is found in the tissues of plants from the Bromeliaceae Family, of which the pineapple (*Ananas comosus* L.) is the best known. This enzyme plays a very important physiological role by intervening in metabolic reactions and protecting the vegetable from the attack of infestations and diseases, also influencing in a special manner nitrogenous metabolism during the flowering stage (Chavez et al., 1998).

### The biochemical properties of bromelain are the following:

Bromelain belongs to the group of cysteine proteases and according to Murachi et al. (1964), among its most important properties are found:
- **Optimal pH,** approximately 7 (Inagami and Murachi, 1963).
- **Stability:** The enzyme maintains its activity on casein at 50° C for 24 hours at a pH range of between 4 to 10.
- **It is stable in solutions of** 25% of methanol (v/v) at 25° C for 20 min, and it loses 50% of its activity when the enzymatic solution is heated to 55° C for 20 min at pH 6 (Whitaker and El-Gharbawi, 1963).
- Lyophilization causes a 27% loss in activity. (Murachi et al., 1964).

**Purity:** Purified by liquid chromatography and the homogeneity of the enzyme was checked via ultracentrifuge, sedimentation, acrylamide gel disk electrophoresis and diffusion analysis. (Murachi and Tachibana, 1966).

The principal amino-terminal residue is valine (Val) and the carboxyl terminal is glycine (Gly). The enzyme is a glycol-protein that contains mannose, xylose, fucose, and N-acetyl-D-glucosamine, it has one oligosaccharide per molecule, which is covalently bonded to the peptide chain (Feinstein and Whitaker, 1964).

Bromelain, obtained from discarded stems, contains one reactive sulfhydryl group per molecule, which is essential for enzymatic catalysis (Murachi and Inagami, 1965). The amino acid sequences proposed on the active site are:
Cys-Gly-Ala-Cys-Trp (Chao and Liener, 1967)
Asn-Gln-Asp-Pro-Cys-Gly-Ala-Cys-Trp (Husain and Lowe, 1968)

### Physical properties of bromelain.

The physical properties of bromelain obtained from stems are indicated; data suggests that the enzyme is a basic protein with a molecular weight of approximately 33,000 Da (Yasuda et al., 1970).

| | |
|---|---|
| Isoelectric Point p1 | 9.55 |
| Absorbency A^{1%}_{cm} at 280 nm | 20.1 |
| Molecular weight | 33.200^{a} 32.100^{b} 33.500^{c} |

| | |
|---|---|
| a: By Sedimentation-Diffusion. b: By intrinsic Sedimentation and Viscosity constant c: By Archibald method (Yasuda et al., 1970). | |

### Conventional Processes of Acquisition.

Currently and conventionally, the process for acquisition of bromelain begins with grinding stems from plants grown in ground to extract and purify the metabolite.

The process for preparing bromelain mentioned in patent US3002891, consists in acquiring bromelain from pineapple stem juice which results in obtaining an enzyme with satisfactory solubility, activity and color in comparison with the extractions made from pineapple juice from fruit.

Patent US3446626 protects a preparation method of a bromelain solution for "antimortem" injection that is administered to animals in order to tenderize the meat. The bromelain is prepared in an injectable solution with a pH of 7.5 and is applied 6 hours before the slaughter of the animal in order to cause tenderizing and softening of the meat.

Patent US3455787 mentions the extraction of bromelain from pineapple stems that involves grinding the stem, extracting the liquid by pressure and centrifuge in order to precipitate fine crystals. Afterwards, the remaining liquid is centrifuged again. While the centrifuge cools, organic solvents and salts, as well as soluble detergents, are used to create the precipitation.

Another manner of acquiring bromelain is mentioned in patent US3699001 in which juice is extracted by submitting the stems to a low temperature treatment, causing a fractioned precipitation from juice clarified with organic compounds and releasing bromelain precipitated with cold organic compounds.

Heinicke and Gortner reported a ketonic method to extract bromelain, in which the stem juice is cooled to 0-4°C, is centrifuged, and the remaining juice is collected, repeating the extraction two times and afterwards vacuum dried and macerated to reduce it to an acetone powder, obtaining 2-5 g of extract for each kilogram of fresh sample. The tests showed that through fractioning with ammonium sulfate less quantity of the protein (0.871g) was obtained but there was more proteolytic activity (1.64 U/mg⁻¹).

Murachi and his collaborators, reported a purification process from raw protein extract from stems, which was suspended in a potassium phosphate buffer with a pH of 6.1 for 30 minutes. The suspension was centrifuged at 5000 rpm for 20 minutes. The precipitate was discarded and the supernatant was divided in different fractions, which were treated differently in order to compare the purity of bromelain obtained.

These processes described, mention that the protein was obtained from plants harvested in the country and they present the disadvantage of being a raw extract, not purified, which causes it to contain various molecules with reduced activity and low extract quality.

### Acquisition Processes by Induction.

This invention considers the use of plants established *in vitro* and stress induced by substances to produce protein. In this respect, work was not found that mentions or expresses a direct induction to obtain pineapple bromelain, it has only been done for purposes of studying the response of the plant to various environmental factors, among which drought is considered to be one of the most harmful environmental phenomenon to human life, which was begun with the identification and molecular characterization of some proteins the appearance of which concurs with stress. As a response to a hydric deficit in the ground caused by sodium chloride, the plants produce a growth regulating molecule, abscisic acid (ABA). This is transported by the xylem from the root to mature leaves where stomata are induced to close in order to avoid in this way a greater loss of water.

There are protein inducers that through hydric stress search for resilience when facing a hydric deficit in plants such as corn in which kinase CK2 is expressed increasing the tolerance of embryos to hydric stress.

As in the case of barley, where proteins 14-3-3 are expressed that act as tolerance regulators when faced with desiccation of the plant. On the other hand, there is another series of work on the induction of proteins by osmotic stress in barley roots. In this case, the treatments with salt induced or increased the synthesis of various proteins, highlighting two of them with a molecular weight of 26 and 27 kD.

In later studies, attention was concentrated on the protein 26 kD known as osmotin. This presents homology with the other proteins involved in the defense mechanisms in plants, like protease inhibitors, corn amyls, and the NP24 protein inducible with salt in the tomato (Singh et al., 1989).

Another protein induction mechanism is from the attack of pathogens or wounds caused to the plant. This is defended using various strategies, such as secondary metabolite synthesis (MS) and the expression of proteins with toxic activities towards the pathogen. An activator of this defense has been AS (salicylic acid) since it has been used successfully on a commercial level in the control and prevention of certain pathogens such as resistance to stress caused by extreme temperatures, the presence of heavy metals and herbicides.

Said resistance is mediated by different RP proteins (resistance proteins), apparently activated through various ways, some dependent on AS, others on jasmonic acid (JA), nitric acid (NO), or ethylene (Leszek S. and Jankiewicz., 2003).

In the case of exogenous application of activating compounds, it is known that each chemical compound induces the expression of different PR proteins. Such is the case of tobacco where the exogenous application of SA gives rise to the induction of defense proteins (PR) such as PR-1, kitinase and b-1,3-gluconase (Leszek S. and Jankiewicz., 2003). However, the previously mentioned inducers do not cause proteins with enzymatic activity.

### DETAILED DESCRIPTION OF THE INVENTION

The main object of this invention is a process for acquiring bromelain with significant proteolytic activity and in greater concentration in the vegetable tissue of the pineapple plant obtained and induced *in vitro,* as compared to the protein generated naturally in the plant in the field and that is extracted using conventional methodologies.

This acquisition process for bromelain using protein inductor substances in pineapple plants *(Ananas comosus),* includes the following stages:

### Selection of the Vegetative Material

*Ananas comosus* plants in the field were selected, which should be between 1 and 2 years of age, 50 cm tall, with a leaf diameter of 90 cm, and that have at least 2 shoots coming from the root, where said shoot is between 15 and 20 cm in height, with a diameter of 4 to 6 cm, weighing between 200 and 500 g, and 5 months old; said characteristics make it possible to select a young shoot that will facilitate the *in vitro* establishment.

### Establishing the Culture

The shoots selected in the previous stage are removed manually, and once in the laboratory, its leaves and rosette are removed from the stem to uncover the apical meristem and then is immediately submerged in a 20% sodium hypochlorite solution for 20 minutes. Afterwards they are taken to a laminar flow bell to perform continuous rinsing with sterile room temperature water, in order to remove sodium hypochlorite residues.

Afterwards those apical meristems that have not be damaged by the sodium hypochlorite are selected to obtain explants measuring 6x6x4 mm, for which cuts are made in the perimeter for the purpose of removing external tissue oxidized by the effects of the sodium hypochlorite.

Said explants are planted in a modified Murashige & Skoog (1962) culture medium, considered for this stage *"Establishment SS".* 1 mg/L of BAP (6- Benzylaminopurine), 30 g/L of sacarose, 2 g/L of gel-rite and with a pH of 5.7 ±0.01 is added.

Finally, the explants planted are incubated in a growth chamber at 27± 2° C, a photo period of 16 hrs/light and 8 hrs/dark for 30 days, where a sprout is obtained measuring 1-2 cm in height with at least four leaves, but said sprouts still does not have axillary sprouts.

### Multiplication of Propagates

A central lengthwise cut is applied to the sprouts from the previous stage (maintaining aseptic conditions), in such a way that two parts are obtained, which are planted *in vitro* in a modified Murashige & Skoog (1962) culture medium, considered for this stage *"Propagation SS".* 4-8 mg/L of BAP (6- Benzylaminopurine), 30 g/L of sacarose, 2 g/L of gel-rite and with a pH of 5.7 ±0.01 is added. These sprouts are incubated in a growth chamber at 27± 2° C, a photo period of 16 hrs/light and 8 hrs/dark for 30 days. During this period each sprout has a production of 4-12 side sprouts which are between 0.5-0.7 cm in height.

This stage is repeated at least once, according to the number of sprouts desired, accordingly the sprouts obtained in this stage are grown under the same propagation conditions as were used for their progenitors. Once the amount of sprouts desired is obtained, the sprouts pass to the next stage which is the induction of bromelain.

### Induction of Bromelain

In this stage a selection is made of the plantlets obtained in the previous stage which are between 1 and 2 cm in height, with an average of 3-5 leaves, and with a weight of 120-250 mg, which are planted *in vitro* in a modified Murashige & Skoog (1962) culture medium, considered for this stage *"Induction SS".* 30 g/L of sacarose, 2 g/L of gel-rite is added and Sacarose (30 - 90g/L) as an osmotic stress inductor.

The sprouts are placed in a growth chamber at 27± 2° C, a photo period of 16 hrs/light and 8 hrs/dark for 7 - 45 days, which is where the sprouts achieve a size of 2-5 cm, a formation of 6-10 leaves and a complete root system.

In the course of this stage it is advisable to evaluate the concentration of the total bromelain (µg protein/g tissue) using the Bradford method, the specific enzyme activity (U/mg protein) using the Dapeau method (1976), at 7, 15, 30, and 45 days in order to discover the effect of the induction period in relation to the production of bromelain. When an enzymatic activity of (5-6 U/mg of protein) is obtained, it is the proper moment to extract the bromelain.

### Extraction of Bromelain

The plants are washed in running water to remove the remains of the culture. Afterwards, they are submitted to a first extraction, passed through a filter press to obtain a first juice that contains between 60 and 80% bromelain, which is stored at (0-5 °C).

The resulting pulp, which obviously contains bromelain, is dissolved in an extraction buffer, that contains cold (0-5° C) potassium phosphate (K₂PO₄) to avoid denaturalization of the enzyme, at a concentration of between 0.03 and 0.2 M, at a pH of between 5.3 and 7.2. This is allowed to repose for 20 to 30 minutes, in order to afterwards pass the mixture through a filter press and obtain a second extraction juice; then the first juice is mixed with the second juice at between 2000 to 5000 rpm for 1-2 minutes, and after centrifuging at 5000-10,000 rpm for 15-20 minutes, at 0-4 °C to remove precipitate and recover the supernatant which is preserved at between -80 and 2 °C and finally it is lyophilized for which said extract should contain at least 10-15% of solids.

### RAW BROMELAIN EXTRACT

One gram of raw extract, obtained by the aforementioned process, is made up of: 0.42 - 3.7 mg ofphenol compounds, 0.9 - 1.038 mg of chlorophyll, and 0.053 - 0.106 µg of total protein.

Therefore, one gram of raw extract has an enzymatic activity of 4.5-6.5 U/mg of protein, (this enzymatic activity has not been found in conventional extracts) and a peroxidase activity of 0.97 - 2.3 U/mg of protein.

The raw extract obtained by using this invention may be used in biotechnical and pharmaceutical applications due to its high enzymatic activity.

Due to the confusion that has originated in the preparation of the Murashige & Skoog (1962) culture medium which is modified from the conventional medium, it is important to point out that the preparation method used starts with a stock solution (Table 1). Starting with this medium the *Establishment SS, Propagation SS,* and the *Induction* SS mediums are prepared.

Therefore, the invention also includes two innovative culture mediums, the *Propagation* and the *Induction* mediums.

**Table 1. Preparation of the Stock Solutions**

| **SOLUTIONS** | **COMPONENTS** | | **AMOUNT** |
|---|---|---|---|
| **Solution A** concentration: 1000X | Volume: 50 ml | Weight | |
| Calcium chloride | CaCl₂-₂H₂O | | 22.0g |
| **Solution B** concentration: 1000X | Volume: 50 ml | | |
| Potassium iodine | KI | | 41.50mg |
| Cobalt chloride | CoCl₂ 6H₂O | | 1.25mg |
| **Solution C** concentration: 400X | Volume: 50 ml | | |
| Monobasic potassium phosphate | KH₂PO₄ | 3.4g | |
| Boric acido | H₃BO₃ | 0.124g | |
| Sodium molybdenum oxide | NaMoO₄ | 0.005g | |
| **Solution D** concentration: 400X | Volume: 50 ml | | |
| Magnesium sulfate | MgSO₄-7H₂O | 7.4g | |
| Magnesium sulfate | MgSO₄-7H₂O | | 0.34g |
| Zinc sulfate | ZnSO₄-7H₂O | 0.172g | |
| Copper sulfate | CuSO₄-5H₂O | | 0.50mg |
| **Solution E** concentration: 200X | Volume: 100 ml | 0.557g | |
| Ferrous sulfate | FeSO₄-7H₂O | 0,745g | |
| Disodium EDTA | Na₂EDTA | | |
| **Solution F** concentration: 100X | Volume: 100 ml | | |
| Glycine | | | 20.0mg |
| Pyridoxine HCl | | 5.0mg | |
| Nicotinic acid | | | 5.0mg |
| Thiamine HCl | | | 1.0mg |
| Myo-Inositol | | | 1.0g |

The modification of the Murashige and Skoog (1962) culture medium consists in that the nitrate solution is not prepared to keep it in stock, but it is prepared when it will be applied in the preparation of the medium, as indicated, in the following medium preparation process from stock solutions.
1. In a precipitation flask place 850 ml of distilled H₂O, add the volume indicated of the concentrated solutions:

**Table 2. Components and amounts of the concentrated solutions.**

| **Concentrated Solutions** | **Volume (ml)** |
|---|---|
| A | 1.0 |
| B | 1.0 |
| C | 2.5 |
| D | 2.5 |
| E | 5.0 |
| F | 5.0 |

2. Weight, add, and stir until the following reagents are dissolved:

**Table 3. Components and amounts of salts in the modified medium SS.**

| | |
|---|---|
| Sacarose | 30g |
| Potassium nitrate (KNO₃) | 1.90g |
| Ammonium nitrate (NH₄NO₃) | 1.65g |

3. Adjust the pH of the medium to 5.7 with sodium hydroxide (NaOH) at 1N or hydrochloric acid (HCl) at 1 N and dilute to 1 liter.
4. Add the gelling agent (gel-rite) 2g/L and heat until it dissolves.
5. Empty 30ml of the conventional SS in 460ml flasks and add growth regulators, sterilize at 121 °C, at a pressure of 1.2 kg/cm² for 15 minutes. At this point the growth regulator in the amounts of 1 mg/L of BAP and SS is called *Establishment* or if 4-8mg/L of BAP is added, it is called *Propagation SS.*
6. If Induction SS is to be prepared, the solution up to point number 4 is made and the inducing substances are added, i.e., sodium chloride (NaCl) at 0.1 - 4.5g/L, sacarose at 40 - 90g/L and salicylic acid at 0.001 - 2.0 mM in combination or separately, once the inducer is added, 30mL is emptied into 460ml flasks and it is sterilized at 121 °C, at a pressure of 1.2 kg/cm² for 15 minutes, except when the medium contains salicylic acid. In this case, the medium is sterilized without the inducer and the inducer is added to the medium once it is sterilized by filtration and under aseptic conditions.

### EXAMPLES

### EXAMPLE 1. BROMELAIN INDUCTION

The objective of this stage was to induce the production of bromelain in pineapple plants through induction substances (sacarose, NaCl, and salicylic acid) with a high enzymatic activity.

In order to perform the evaluation of the inducers, a factor design was carried out of 3x3x3 with three inducers at three different concentrations and three monitoring times (Table 4), for each treatment The evaluations are carried out using the inducers separately or in combination giving a total of 64 treatments.

**Table 4. Statistical design for the bromelain inducers.**

| **Factors** | | **Levels** | |
|---|---|---|---|
| Control | 0 | 0 | 0 |
| Salicylic Acid (mM) | 0.01 | 0.1 | 1.0 |
| NaCl (g) | 0.5 | 1.5 | 2.5 |
| Sacarose (g) | 30 | 60 | 90 |
| Time (days) | 15 | 30 | 45 |

The parameters evaluated are the concentration of the total protein (µg protein/g tissue) using the Bradford method, the specific enzyme activity (U/mg protein) using the Dapeau method (1976), at 7, 15, 30, and 45 days in order to discover the effect of the induction period.

The induction was begun with the selection of the sprouts previously established *in vitro* with a size of 2 cm and an average of 3 leaves and weighing 120 mg. These plants were planted in a *Induction* SS without growth regulator and adding sodium chloride NaCl at 0.1 - 4.5 g/L as a hydric stress inductor, sacarose at 30 - 90 g/L. as an osmotic stress inducer and salicylic acid at 0.001 - 2.0 mM as an inducer of resistance to pathogens, said inducer substances are added in combination (two or more inducers) or separately (one single inducer) following the treatment (Table 5).

**Table 5. Treatments for bromelain induction**

| **Treatments for the induction of bromelain** | |
|---|---|
| Zl=Modified SS (no inducer)* | Z33= Modified SS + NaCl (1.5g/L) + SAC (60 g/*L)* |
| Z2= Modified SS + SA (0.01mM) | Z34= Modified SS + NaCl (1.5g/L) + SAC (90 g/L) |
| Z3= Modified SS + SA (0.1mM) | Z35= Modified SS + NaCl (2.5 g/L) + SAC (30 g/L) |
| Z4= Modified SS + SA (1.0mM) | Z36= Modified SS + NaCl (2.5g/L) + SAC (60 g/L) |
| Z5= Modified SS + NaCl (0.5g/L) | Z37= Modified SS + NaCl (2.5g/L) + SAC (90 g/L) |
| Z6= Modified SS + NaCl (1.5 g/L) | Z38= Modified SS + SA (0.01mM) + NaCl (0.5g/L) + SAC (30 g/L) |
| Z7 =Modified SS + NaCl (2.5 g/L) | Z39= Modified SS + SA (0.01mM) + NaCl (0.5g/L) + SAC (60 g/L) |
| Z8 = Modified SS + SAC(30 g/L) | Z40= Modified SS + SA (0.01mM) + NaCl (0.5g/L) + SAC (90 g/L) |
| Z9 = Modified SS + SAG (60 g/L) | Z41= Modified SS SA (0.01mM) + NaCl (1.5g/L) + SAC (30 g/L) |
| Z10= Modified SS + SAC (90 g/L) | Z42= Modified SS + SA (0.01mM) + NaCl (1.5g/L) + SAC (60 g/L) |
| Z11= Modified SS + SA (0.01mM) + NaCl (0.5g/L) | Z43= Modified SS + SA (0.01mM) + NaCl (1.5g/L) + SAC (90 g/L) |
| Z12= Modified SS + SA (0.01mM) + NaCl (1.5g/L) | Z44= Modified SS + SA (0.01mM) + NaCl (2.5g/L) + SAC (30 g/L) |
| Z13= Modified SS + SA (0.01mM) + NaCl (2.5g/L) | Z45= Modified SS + SA (0.01mM) + NaCl (2.5g/L) + SAC (60 g/L) |
| Z14= Modified SS + SA (0.1mM) + NaCl (0.5g/L) | Z46= Modified SS + SA (0.01mM) + NaCl (2.5g/L) + SAC (90 g/L) |
| Z15= Modified SS + SA (0.1mM) + NaCl (1.5g/L) | Z47= Modified SS + SA (0.1mM) + NaCl (0.5g/L) + SAC (30 g/L) |
| Z16= Modified SS + SA (0.1mM) + NaCl (2.5g/L) | Z48= Modified SS + SA (0.1mM) + NaCl (0.5g/L) + SAC (60 g/L) |
| Z17= Modified SS + SA (1.0mM) + NaCl (0.5g/L) | Z49= Modified SS + SA (0.1mM) + NaCl (0.5g/L) + SAC (90 g/L) |
| Z18= Modified SS + SA (1.0mM) + NaCl (1.5g/L) | Z50= Modified SS + SA (0.1mM) + NaCl (1.5g/L) + SAC (30 g/L) |
| Z19= Modified SS + SA (1.0mM) + NaCl (2.5g/L) | Z51= Modified SS + SA (0.1mM) + NaCl (1.5g/L) + SAC (60 g/L) |
| Z20= Modified SS + SA (0.01mM) + SAC (30 g/L) | Z52= Modified SS + SA (0.1mM) + NaCl (1.5g/L) + SAC (90 g/L) |
| Z21= Modified SS + SA (0.01mM) + SAC (60 g/L) | Z53= Modified SS + SA (0.1mM) + NaCl (2.5g/L) + SAC (30 g/L) |
| Z22= Modified SS + SA (0.01mM) 4- SAC (90 g/L) | Z54= Modified SS+ SA (0.1mM)+ NaCl (2.5g/L)+ SAC (60 g/L) |
| Z23= Modified SS + SA (0.1mM) + SAC (30 g/L) | Z55= Modified SS + SA (0.1mM) + NaCl (2.5g/L) + SAC (90 g/L) |
| Z24= Modified SS + SA (0.1mM) + SAC (60 g/L) | Z56= Modified SS + SA (1.0mM) + NaCl (0.5g/L) + SAC (30 g/L) |
| Z25= Modified SS + SA (0.1mM) + SAC (90 g/L) | Z57= Modified SS + SA (1.0mM) + NaCl (0.5g/L) + SAC (60 g/L) |
| Z26= Modified SS + SA (1.0mM) + SAC (30 g/L) | Z58= Modified SS + SA (1.0mM) + NaCl (0.5g/L) + SAC (90 g/L) |
| Z27= Modified SS + SA (1.0mM) + SAC (60 g/L) | Z59= Modified SS + SA (1.0mM) + NaCl (1.5g/L)+ SAC (30 g/L) |
| Z28= Modified SS + SA (1.0mM) + SAC (90 g/L) | Z60= Modified SS + SA (1.0mM) + NaCl (1.5g/L) + SAC (60 g/L) |
| Z29= Modified SS + NaCl (0.5g/L) +SAC (30 g/L) | Z61= Modified SS + SA (1.0mM) + NaCl (1.5g/L) + SAC (90 g/L) |
| Z30= Modified SS + NaCl (0.5g/L) +SAC (60 g/L) | Z62= Modified SS + SA (1.0mM) + NaCl (2.5g/L) + SAC (30 g/L) |
| Z31= Modified SS + NaCl (0.5g/L) + SAC (90 g/L) | Z63= Modified SS + SA (1.0mM) + NaCl (2.5g/L) + SAC (60 g/L) |
| Z32= Modified SS + NaCl (1.5 g/L) + SAC (30 g/L) | Z64= Modified SS + SA (1.0mM) + NaCl (2.5g/L) + SAC (90 g/L) |

| | |
|---|---|
| ***treatment without the application of inducer substances, used as a test control to verify the effect of the inducers on the production of bromelain.** **SA: Salicylic Acid. SAC: Sacarose. NaCl: (Sodium Chloride)** | |

Once the inducers are added to the modified SS it is sterilized at 121 °C, at a pressure of 1.2 kg/cm² for 20 minutes. In the treatments that contain salicylic acid, the modified SS medium is first sterilized and later the acid is added, which was sterilized through filtration, under aseptic conditions in a laminar flow bell. Here it is advisable to add the acid to the medium before it is cooled to achieve the homogenization of the inducer with the medium before it is solidified.

The sprouts are planted under aseptic conditions in 460 ml flasks that contain 30 ml of the *Induction SS,* said flasks are closed and sealed with clear tape in order to avoid any filtering of air and with the air, contamination of same.

The treatments are moved to a growth room under controlled conditions at 27± 2° C and a photoperiod of 16 hr/light and 8 hr/darkness for a period of 7, 15, 30 and 45 days.

Once the first 7 days of induction have passed, enzymatic extraction is carried out in order to obtain raw extract. Three 30 µg/ml samples are taken for each treatment to determine in triplicate the specific activity.

During the evaluation of the inducers it can be concluded that all the inducers applied present statistical differences, e.g., all the treatments to which an inducer substance was applied showed a positive effect in producing bromelain in greater enzymatic activity than the treatments without inducers (controls). Table 6 shows the differences in the treatment means that resulted to be statistically significant, e.g., those that presented a maximum of specific activities during 15, 30 and 45 days.

**Table 6. Effects of the inducers on the production of bromelain.**

| **Treatment** | **Total Protein** **(µg protein/g tissue)** Differences in means (15 days) Tukey 95% | **Specific Enzyme** **(U/mg protein**) Differences in means (15 days) Tukey 95% |
|---|---|---|
| **Z1** Control | 36.2628 | 1.0057 |
| **Z4** 15 days | 71.0326 | 3.1718 |
| **Z7** 15 days | 56.1715 | 4.9846 |
| **Z9** 15 days | 52.9024 | 6.3550* |
| **Z4** 30 days | 106.5849 | 2.3413 |
| **Z7** 30 days | 106.8685 | 2.2570 |
| **Z9** 30 days | 112.4086 | 4.9877 |
| **Z4** 45 days | 158.0485 | 3.1131 |
| **Z7** 45 days | 118.9159 | 2.6469 |
| **Z9** 45 days | 105.4586 | 5.1257 |

| | | |
|---|---|---|
| All the means presented statistical differences (Tukey 95%). * Statistically important mean. | | |

The behavior of the inducers with regard to time is the following.
At 7 days there is a significant presence of the enzyme. At 15 days the production of bromelain increases. At 30 days it decreases and at 45 days it increases.

### Following is the assumption:

When the plant discovers that it is being threatened it produces defense proteins, in this case, bromelain which gives rise to the assumption that it is a rapid response protein due to presenting enzymatic activity at 7 days after induction and presents its maximum specific activity at 15 days. After 30 days, the plants adapted to their conditions and in turn produced bromelain in lower quantities. At 45 days after induction, the plants were submitted to stress due to a shortage of nutrients in the medium and they began to produce bromelain again, but in lower quantities than the amounts at the beginning.

### EXAMPLE 2. PROCEDURE FOR BROMELAIN EXTRACTION

The plantlets grown *in vitro* in different culture mediums indicated in this invention were taken and rinsed in running water to remove the *Induction* SS medium. 330 g of plants were weighed, placed in an extractor to be ground and 200 ml of juice was obtained (first extraction) that was stored at a temperature of 4 °C. 670 ml of extraction buffer (monobasic potassium phosphate and dibasic potassium phosphate) is added to the pulp obtained from the extraction at a pH of 6.1 and a concentration of 0.05M; this is allowed to repose for 20 minutes in order to afterwards collect the enzyme and pass it through an extractor and thus obtain the second juice. The following step is to mix and homogenize the juice from the first extraction with the second extraction for 1 minute at 2000 rpm and to then centrifuge at 10000 rpm for 20 min. at 4 °C, to afterwards recover the supernatant with a volume of approximately 800 ml which is frozen at -80 °C and lyophilized (10% of solids) for its preservation.

The extraction procedure is applied for all the treatments of which the amount of protein present is determined (Bradford) expressing the protein per ml of extract in mg. A standard curve is used for the protein tests. The standard used was bovine serum albumin at 0.20-1.0 mg/ml.

Based on the results, the following specific activities were obtained compared with imported commercial bromelain and bromelain induced from *in vitro* plants.

**Table 7. Table of the comparison of the specific activity obtained from different methods**

| **Treatments to obtain bromelain (30 mg/ml protein sample)** | **Specific activity (U/mg⁻¹ protein)** |
|---|---|
| **Sacarose (15 days from induction)** | **635** |
| **NaCl (15 days from induction)** | **4.98"** |
| **Salicylic acid (15 days from induction)** | **3.17** |
| **Commercial bromelain for exportation** | **1.74** |
| **Patent CU22515** | **1.54** |
| **Plants without *in vitro* induction** | **1.00** |

Upon performing various induction tests for the production of bromelain, greater specific activity is obtained using sacarose as an inducer in a range of 30-90 g/L; in this case the addition of sacarose to the medium caused osmotic stress in the plant which used the production of bromelain as a natural response.

With the use of the inducers in the *in vitro* propagation of pineapple plants a greater enzymatic activity is generated ranging from 200 up to 600% in comparison with the bromelain produced naturally in conventionally grown pineapple plants.

The bromelain extracted through this process do not require purification methods to increase its specific proteolytic activity which is now greater than the proteolytic activity of bromelain obtained by current extraction methods that include a purification stage.

Not submitting the bromelain to a purification process makes its production much more profitable and minimizes the losses of raw material which occur during the purification process.

### BIBLIOGRAPHY

1. Chao L.P. and I.E. Liener. 1967. Biochem. Biophys. Res. Commun. 27: 100.
2. Chavez P. M., Marquez P. M., Hernandez, Rodriguez A.G. and B. Santos. 1998. "Inventor's Certificate". Office of Cuban Industrial Property.
3. Feinstein G. and J.R. Whitaker. 1964. Biochemistry 3:1-050.
4. Felton G.E. 1980. Fibrinolytic and antithrombotic action of bromelain may eliminate thrombosis in heart patients. Med Hypotheses; 6:1123-1133.
5. Fernandez G. M. 200-1. Bromelain: The Useful Side of Eating Pineapples. Cellular Biology Unit. University of Chile.
6. Heinicke R.M. y W.A. Gortner. 1957 Econ. Bot. 11: 225.
7. Husain S.S. and G. Lowe. 1968, Chem. Commun. p: 1387.
8. Inagami T. and T. Murachi. 1963. Biochemistry 2:1439.
9. Leszek S. and Jankiewicz. 2003. Growth, development, and resistance regulators in plants. Properties and acction. (1):321-350.
10. MacKay Douglas ND and Miller Lan L. 2003. Nutritional support for wound healing. Alternative Medicine. Review. 8: 359-377.
11. Murachi T. and T. Inagami. 1965. Biochemistry 4: 2815.
12. Murachi T. and A. Tachibana. 1966. Biochemistry 5: 2756.
13. Murachi T., Yasui M. and Y. Yasuda. 1964. Biochemistry. 3:48.
14. Murashige T. and F. Skoog. 1962. A revised medium for rapid growth and bioassays with tobacco culture. Phisiol. Plant. 15: 473.
15. Singh, N.K., Handa, A.K., Hasegewa, P.M. and Bressan, R.A. 1989. Molecular cloning of osmotin and regulation of its expression by ABA and adaptation to low water potential. Plant Physiol., 90:1096-1101.
16. Yasuda Y.N. Takahashi and T. Murachi. 1970. Biochemistry 9:25.
17. Whitaker J. R. and M. El-Gharbawi. 1963. Biochemistry 2:476.

Having described my invention in a sufficiently clear manner, I feel that it is an innovation and therefore, claim the content of the following clauses as my exclusive property:

## Claims

1. Method for obtaining bromelain from pineapple plants according to Clause 1, **characterized in that** it includes the following steps:
A. Selection of the Vegetative Material Select *Ananas comosus* plants in the field, which should be between 1 and 2 years of age, 50 cm tall, with a leaf diameter of 90 cm, and that have at least 2 shoots originating from the root where said shoot should be 15 to 20 cm tall with a diameter of 4-6 cm, and a weight of 200-500 g and 5 months of age.
B. Establishing the Culture. Remove the shoot manually, and once in the laboratory, remove its leaves and rosette from the stem to uncover the apical meristem; perform disinfection of the central meristem by submerging it in a 20% sodium hypochlorite solution for 20 minutes. Afterwards they are taken to a laminar flow bell to perform continuous washings with sterile room temperature water, in order to remove sodium hypochlorite residues; select those apical meristems that have not be damaged by the sodium hypochlorite to obtain explants measuring 6x6x4 mm, for which cuts are made in the perimeter for the purpose of removing external tissue oxidized by the effects of the sodium hypochlorite; plant said explants in a modified Murashige & Skoog (1962) culture medium, considered for this stage *"Establishment SS".* 1 mg/L of BAP (6- Benzylaminopurine), 30 g/L of sacarose, 2 g/L of gel-rite and with a pH of 5.7 ±0.01 is added; incubate the explants in a growth chamber at 27± 2° C, a photo period of 16 hr/light and 8 hr/dark for 30 days, where a sprout is obtained measuring 1-2 cm in height with at least four leaves, but said sprouts should still not have axillary sprouts;
C. Multiplication of Propagates Make a central lengthwise cut on the previously mentioned sprouts so as to obtain two parts; plant the parts obtained from the cut in a modified Murashige & Skoog (1962) culture medium, considered for this stage *"Propagation SS".* 4 mg/L of BAP (6- Benzylaminopurine), 30 g/L of sacarose, 2 g/L of gel-rite and adjusted to a pH of 5.7 ±0.01 is added; incubate the sprouts in a growth chamber at 27± 2° C, a photo period of 16 hrs/light and 8 hrs/dark for 30 days. During this period each sprout has a production of 4-12 side sprouts which are between 0.5-0.7 cm in height; plant the sprouts again (maintaining aseptic conditions) according to the number of sprouts desired, accordingly, the sprouts obtained in this stage are grown under the same propagation conditions as were used for their progenitors;
D. Induction of Bromelain. Select plantlets obtained in the previous stage, which are between 1 and 2 cm in size with an average of 3-5 leaves and a weight of 120-250 mg; plant the plantlets selected in a modified Murashige & Skoog (1962) culture medium, considered for this stage *"Induction SS".* 30 g/L of sacarose, 2 g/L of gel-rite and sacarose (30 - 90g/L) as an osmotic stress inducer is added; incubate the sprouts in a growth chamber at 27± 2° C, a photo period of 16 hrs/light and 8 hrs/dark for 7 - -45 days, which is where the sprouts achieve a size of 2-5 cm, a formation of 6-10 leaves and a complete root system; and
E. Extraction of Bromelain. Extract the bromelain when an enzymatic activity of (5-6 U/mg of protein) is obtained; submit the plantlets to a first extraction, passing them through a filter press to obtain a first juice that contains between 60 and 80% bromelain, which is stored at (0-5 °C); suspend the resulting pulp that obviously still contains bromelain in an extraction buffer that contains cold (0-5 °C) potassium phosphate (K₂PO₄) to avoid denaturalization of the enzyme, at a concentration of 0.03 to 0.2 M, with a pH of between 5.3 and 7.2, allowing it to repose for 20 to 30 minutes, and afterwards pass the mixture through the filter press to obtain a second juice from this extraction; mix and homogenize the first juice with the second juice at 2000-5000 rpm for 1-2 minutes, and afterwards centrifuge it at 5000 - 10,000 rpm for 15 - 20 minutes at 0-4 °C to remove precipitate and recover the supernatant which is preserved at -80 to 0 °C; lyophilize the extract which should contain at least 10-15% of solids.

2. Culture medium for the propagation of *in vitro* pineapple plants *(Ananas comosus)* that includes: a 99% modified Murashige & Skoog (1962) culture medium, and 1% of 4-8 mg/L of BAP.

3. Culture medium for the induction of *in vitro* pineapple plantlets *(Ananas comosus)* that is made up of: a 99% modified Murashige & Skoog (1962) culture medium, and at least 1% of an induction substance such as sacarose (30-90 g/L); or sodium chloride (0.1 to 4.5 g/L); or salicylic acid (0.001-2.0 mM).

4. A raw extract of pineapple plantlets *(Ananas comous),* **characterized in that** it is obtained by the method described in Clause 1.

5. A raw extract from pineapple plantlets *(Ananas comous),* according to Clause 4, **characterized in that** it contains in one gram: 0.42 - 3.7 mg of phenol compounds, 0.9 - 1.038 mg of chlorophyll, and 0.053 - 0.106 µg [sic].

6. A raw extract from pineapple plantlets *(Ananas comous),* according to Clause 5, **characterized in that** it contains an enzymatic activity of 4.5 - -6.5 U/mg of protein and a peroxidase activity of 0.97 - 2.3 U/mg of protein.
